# EUROPEAN PATENT APPLICATION

(11) **EP 1 875 930 A1**
(43) Date of publication of application: **09.01.2008**
(21) Application number: 07252542.1
(22) Date of filing: 22.06.2007
(51) Int. Cl.: A61L 26/00, A61F 13/00

(54) **Wound pad**

(30) Priority: 07.07.2006 US 819152 P
(71) Applicant: Tyco Healthcare Group LP, Norwalk, Connecticut 06856 (US)
(72) Inventor: Wenchell, Thomas, Durham, CT 06422 (US)
(74) Representative: Maschio, Antonio

(57) **Abstract**

The present disclosure provides tissue supports which may be utilized by themselves or in combination with other tissue closure means to enhance wound closure and healing. The tissue supports are made of a material which shrinks upon application of heat orlight-to further support the tissue and/or wound to which it is applied.

## Description

### CROSS-REFERENCE TO RELATED APPLICATIONS

This application claims the benefit of and priority to U.S. Provisional Patent Application No. 60/819,152 filed July 7, 2006, the entire disclosure of which is incorporated by reference herein.

### TECHNICAL FIELD

The present disclosure provides a tissue support for use in connection with wound closure methods. The tissue support may be utilized by itself or in combination with surgical staples, sutures, adhesives, and the like to close a wound. Methods for sealing wounds with the tissue supports of the present disclosure are also provided.

### BACKGROUND

A variety of techniques have been used to bond or seal tissue. For example, different types of tissues have been mechanically bound or sealed utilizing a number of procedures, materials and methods including sutures, staples, tapes and/or bandages. In some applications, these materials are made of absorbable materials which are intended to bond and/or seal tissue as it heals and then are absorbed by the body over a period of time.

Other methods for sealing tissue include the use of tissue adhesives. One type of adhesive that is currently available is a cyanoacrylate adhesive. However, there is the possibility that a cyanoacrylate adhesive can degrade to generate undesirable by-products such as formaldehyde. Another disadvantage with cyanoacrylate adhesives is that they can have a high flexural modulus which can limit their usefulness.

Another type of tissue sealant that is currently available utilizes components derived from-bovine and/or human sources. For example, fibrin sealants are available. However, as with any natural material, variability in the material is frequently observed and, because the sealant is derived from natural proteins, there may be viral transmission concerns.

Thus, wound closure means, including those capable of augmenting other methods including staples and sutures, remain desirable.

### SUMMARY

The present disclosure provides materials suitable for use as tissue supports for wounds and methods for sealing wound sites with said materials. In embodiments, a method for sealing a wound site may include providing a sheet of a bioabsorbable polymeric material, applying said sheet of polymeric material to a-wound site, applying energy to said sheet of polymeric material, and-shrinking said sheet of polymeric material around said wound site.

Suitable bioabsorbable polymeric materials which may be utilized to secure a wound site by shrinking around the wound site include, for example, glycolide, glycolic acid, lactide, lactic acid, caprolactone, dioxanone, trimethylene carbonate, dimethyl trimethylene carbonate, homopolymers thereof, copolymers thereof, and combinations thereof.

Energy which may be utilized to shrink the polymeric material includes, for example, heat, light, magnetism, ultrasound, and combinations thereof. In some embodiments, heat from the body of a patient to which the tissue support is applied may be utilized to induce-shrinking. In other embodiments, the addition of saline, water, body fluids, combinations-thereof, -and the like, may be applied to the polymeric material to induce shrinking.

Various wounds may be treated in accordance with the methods and materials of the present disclosure. In embodiments, the wound site may be an anastomosis. In other embodiments, the methods and materials of the present disclosure may be utilized in combination with other wound closure means such as sutures, staples, adhesives, and combinations thereof.

The bioabsorbable polymeric material utilized in the present disclosure may, in embodiments, also contain a medicinal agent. In embodiments, the bioabsorbable polymeric material may be fully degraded from about one day to-about six months after application.

### BRIEF DESCRIPTION OF THE DRAWINGS

FIG. 1 is a cross sectional view of a tissue support of the present disclosure joining two sections of bowel; and

FIG. 2 is a depiction of tissue supports of the present disclosure supporting staple lines closing incisions remaining after a gastric bypass.

### EMBODIMENTS

The present disclosure provides tissue supports for closing wounds. The tissue supports may be utilized by themselves or, in embodiments, may be utilized in combination with other wound treatments such as staples, sutures, adhesives, and the like. The tissue supports are made of biocompatible materials which, in embodiments, may be bioabsorbable.

Suitable biocompatible materials which may be used to form a tissue support of the present disclosure include, but are not limited to, absorbable polymers made from glycolide, glycolic acid, lactide, lactic acid, caprolactone, dioxanone, trimethylene carbonate and dimethyl trimethylene carbonate. Homopolymers, copolymers, (block or random) and combinations including mixtures and blends of such polymers and/or copolymers may also be utilized. In some embodiments, the tissue support may be made of a polymer such as polycaprolactone, polylactic acid, polydioxanone, polytrimethylene carbonate, and/or combinations thereof.

The tissue support may be in any suitable-shape. In embodiments, the tissue support may be a flat-sheet or film. In other embodiments the tissue support may possess a tubular shape. Regardless of the configuration, a tissue support of the present disclosure may have a thickness of from about 0.002 inches to about 0.05 inches, in embodiments from about 0.01 inches to about 0.03 inches. A flat sheet or tubular shape will permit the tissue support to be placed over or wrapped around tissue surrounding a wound, including surgical incisions and anastomoses.

In embodiments, the tissue support may shrink after application to tissue, thereby further supporting and closing any wound to which it is applied and minimizing any air or blood leaks from the wound. Means for inducing shrinkage of the tissue support are within the purview of those skilled in the art and include, for example, the application of heat including body heat, radiation including light and ultraviolet (UV) radiation, magnetism, ultrasound, saline, water, body fluids, combinations-thereof, and the like.

In some embodiments, the body heat of a patient (about 37° C) to which the tissue support of the present disclosure is applied may be sufficient to shrink the tissue support. In other embodiments, external sources of heat may be applied to shrink the tissue support. Heating can be accomplished by using a gas or liquid heating medium, heating devices, ultrasonic waves, or the like. Heating may be at a temperature from about 30° C to about 50° C, in embodiments from about 39° C to about 43° C (just above human body temperature). Heat may be applied for a period of time from about one second to about ten minutes, in embodiments from about thirty seconds to about two minutes. Of course, care should be taken to utilize a heating temperature which will not burn the tissue of the patient to which a tissue support of the present disclosure has been applied. When a liquid heating medium is used, physiological saline solution or alcohol may be desirable.

In still other embodiments, light having a wavelength in the visible region, from about 400 nm to about 700 nm, may be applied to the tissue support to induce shrinking of the tissue support thereby sealing around the tissue to which it is applied. In other embodiments, light in the UV region from about 0.3 µm to about 3 nm may be applied to the tissue support to induce shrinkage of the tissue support thereby sealing around the tissue to which it is applied. Light may be applied for a period of time from about one second to about ten minutes, in embodiments from about two minutes to about four minutes.

While not wishing to be bound by any theory, it is believed the materials utilized to form the tissue support undergo polymeric coiling upon the application of heat or light energy, which thereby causes the tissue support to shrink. The amount of shrinkage may vary depending upon the materials utilized to form the tissue support and the means utilized to induce shrinking. In embodiments, a tissue support of the present disclosure may shrink from about 5% to about 50% in area, in embodiments from about 15% to about 30% in area.

The shrinkage of the tissue support of the present disclosure may enhance the adherence of the tissue support to tissue, or itself where the tissue support is in a tubular configuration or wrapped around a tubular organ such as the bowel. Where placed over the skin or a non-tubular organ, the tissue support may itself adhere to the tissue surrounding the-wound site, thereby drawing the wound together as the tissue support shrinks.

The tissue support maybe solid or semi-permeable, enabling oxygen transport to/from the wound site. The tissue support may also be perforated or possess pores to further enhance oxygen permeability of the support. Pores in the tissue support may be from about 2.5 mm to about 2.5x10⁻⁷ mm in diameter, in embodiments from about 1 mm to about 1x10⁻⁵ mm in diameter.

The degradation rates of the polymers utilized to form the tissue support may be varied depending upon the length of time needed to support the tissue repair. In embodiments, tissue supports of the present disclosure may be fully degraded after a time -period from about one day to about six months after application, in other embodiments from about two days to about fourteen days after application.

The tissue supports of the present disclosure seal wounds in tissue to prevent or control leaks of fluids such-as air or blood, thereby enhancing healing of a wound or surgical site. In some embodiments, the biocompatible tissue support of the present disclosure can be used to bind tissue together either as a replacement of, or as a supplement to, sutures, staples, biocompatible adhesives, tapes, bandages, and the like.

Tissue supports of the present disclosure may be utilized to enhance the sealing and healing of any wound. In embodiments, the tissue supports may be utilized to enhance the sealing and healing of a surgical incision. The wound or incision may be located on the skin of a patient. In other embodiments, the wound or incision may be internal. Such internal wounds may be the result of the removal of an organ or any trauma whereby sutures, staples, adhesives, and the like may be utilized to close and seal the wound site.

The biocompatible tissue support of the present disclosure can be used in surgery to prevent or inhibit bleeding or fluid leakage both-during and after a surgical procedure. It can also be applied to prevent air leaks associated with pulmonary surgery. The biocompatible tissue support may be applied-directly to the desired area to seal off any fluid or air movement.

For example, staple lines and/or suture lines may be prone to leak air or fluids such as blood, which can produce complications in a patient undergoing an operation. The use of a tissue support of the present disclosure in combination with surgical staples may minimize and/or prevent the leakage of any air or fluids from the staple line. In addition, the use of a tissue support of the present disclosure may eliminate or reduce the number of sutures and/or staples otherwise required to close a wound. The biocompatible tissue support can thus be particularly suitable for use with delicate tissues where sutures, staples, clamps or other conventional tissue closure mechanisms may cause further tissue damage.

Alternatively, the tissue support of the present disclosure may be utilized in combination with a biocompatible adhesive to close a wound. Any biocompatible adhesive within the purview of one skilled in the art may be utilized. In such an embodiment, the adhesive may not only help close the wound, but may also help the tissue support adhere to the tissue adjacent the wound.

In other embodiments, tissue supports of the present disclosure may be utilized in combination with sutures or staples to seal an anastomosis. Anastomosis is the surgical joining of separate hollow organ sections so that the sections intercommunicate with each other. Typically, the anastomosis procedure follows surgery in which a diseased or defective section of hollow tissue is removed and the remaining end sections are to be joined. Depending on the desired anastomosis procedure, the end sections may be joined by either circular, end-to-side or side-to-side organ reconstruction methods.

Examples of anastomosis procedures include those utilized in repair of the bowel and those utilized with vascular repair. Apparatus for use in such procedures are also known. For example, circular staplers and linear stapling and cutting instruments are well known in the art for the anastomosis of bowel tissue. Circular staplers are used primarily for end-to-end anastomosis of bowel tissue. In a circular anastomosis procedure, the two ends of the organ sections are joined by means of a stapling instrument which drives a circular array of staples through the end sections and simultaneously cores any overlapping tissue to free the tubular passage. Circular staplers also have been used for the end-to-side type of anastomosis on large organs such as bowel. A tissue support of the present disclosure may be utilized in combination with sutures-or staples in the repair of an anastomosis of the bowel to minimize air and/or fluid leakage from a suture or staple line.

In vascular anastomosis, it is often desirable to join the graft vessel to the target vessel at an acute angle to facilitate laminar blood flow through the joined vessels, or to avoid kinking of the graft vessel. Also, the size of the passageway between the joined vessels is limited by the diameter of the graft vessel. For a side-to-side anastomosis, the passageway size can be large relative to the vessel diameters by choosing the appropriate length of staple joining lines. A number of devices for augmentation of the suturing techniques for vascular anastomosis have been developed. These devices attempt, with varying degrees of success, to reduce the difficulty in repeatedly passing a needle and thread through the blood vessel walls. Examples include those found in U.S. Patent Nos. 5,571,090, 4,803,984, and 5,545,148. A tissue support in accordance with the present disclosure may be utilized around a staple or suture line-utilized in a vascular anastomosis to minimize blood loss from the staple or suture line.

In other embodiments, anastomosis repair methods may be utilized to perform a gastric bypass procedure. Such methods include, for example, those set forth in U.S. Patent Application Publication No. 2004/0087977, the entire disclosure of which is incorporated by reference herein. A tissue support of the present disclosure may be utilized in combination with sutures and/or staples in the performance of a gastric bypass procedure regardless of the location of the gastric bypass, that is, the pharynx, esophagus, stomach, duodenum, colon, and the like.

Alternatively, the tissue support of the present disclosure may be utilized in combination with a biocompatible adhesive to close a wound. In such an embodiment, the adhesive may not only help close the wound but may also help the tissue-support adhere to the tissue adjacent the wound.

In another embodiment, the biocompatible tissue support can be used to attach skin grafts and position tissue flaps during reconstructive surgery.

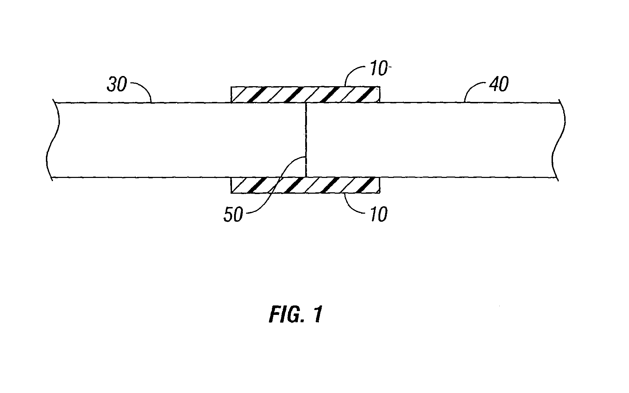

Application of the biocompatible tissue support can be done by any conventional means. These include direct manipulation of the tissue support on the tissue surface. In open surgery, application by hand, forceps-or the like is contemplated. Where the wound site permits, the tissue support may be in a tubular form and placed around the site to be repaired. For example, with an end to end anastomosis of the bowel, the desired section of boweL may be removed, and a tissue support of the present disclosure may be placed over the anastomosis so that the tissue support overlaps the joined ends. Such a repair is depicted in Figure 1, which is a cross sectional view of tissue support 10 joining two sections of bowel, 30 and 40 at junction 50. The tissue support may be utilized by itself to join the free ends-of the bowel or may be used in combination with other means for joining the free ends, including staples, sutures, adhesives, and the like.

In some embodiments, the tissue support may be tubular. In such a case, the tubular support may be placed over a wound and shrunk as described above to enhance wound closure. In some embodiments, one end of a tubular support may be placed over one free end of a bowel, a second end of the tubular tissue support may be placed over a second free end of the bowel, and the tissue support may then be shrunk to join the two ends of the bowel, optionally in combination with other closure means described above. In other embodiments, the tissue support of the present disclosure may be in a sheet or film configuration and placed over or wrapped around the wound site to be treated. Thus, the sheet or film could be wrapped around the bowel at the site of an anastomosis or, alternatively, placed over a wound or incision.

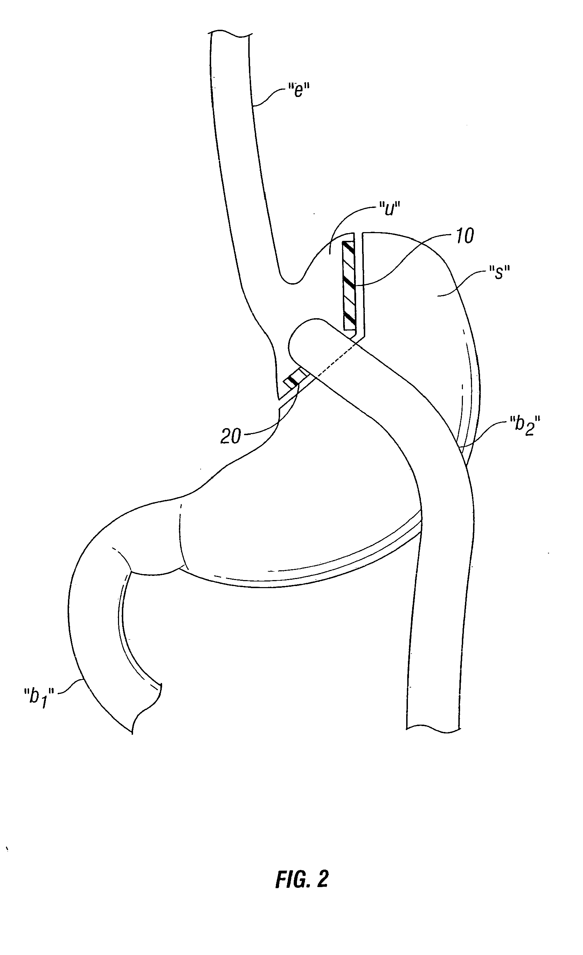

For example, in some embodiments, a tissue support of the present disclosure may be utilized to further close incisions resulting from a gastric bypass. A detailed description of an apparatus and method for performing a bypass procedure of a digestive system is described in U.S. Patent Application Publication No. 2004/0087977, the entire disclosure of which is incorporated by reference herein. An example of the use of tissue supports of the present disclosure in connection with such a procedure is depicted in Figure 2. As set forth in Figure 2, tissue supports 10 and 20 may be utilized to further support staple lines closing incisions remaining after upper-stomach "u", which is adjacent esophagus "e", has been separated from the remainder of stomach "s" and bowel section "b2" has been joined with upper stomach "u" after separation of bowel section "b2" from bowel section "b1".

Biologically active agents may be combined with the polymers utilized to form the tissue supports of the present disclosure. For example, naturally occurring polymers, including proteins such as collagen and derivatives of various naturally occurring polysaccharides such as glycosaminoglycans, can be combined with the polymers utilized to form tissue supports of the present disclosure.

A variety of optional ingredients including medicinal agents may also be combined with the polymers utilized to form tissue supports of the present disclosure. The term "medicinal agent", as used herein, is used in its broadest sense and includes any substance or mixture of substances that have clinical use. Consequently, medicinal agents-may or may not have pharmacological activity per se, e.g., a dye. Alternatively a medicinal agent could be any agent which provides a therapeutic or prophylactic effect, a compound that affects or participates in tissue growth, cell growth, cell differentiation, a compound that may be able to invoke a biological action such as an immune response, or could play any other role in one or more biological processes.

Examples of classes of medicinal agents which may be utilized in accordance with the present disclosure include antimicrobials; analgesics; antipyretics; anesthetics; antiepileptics; antihistamines; anti-inflammatory agents such as hormonal agents; hydrocortisone, prednisolone, prednisone, non-hormonal agents, allopurinol, indomethacin, phenylbutazone and the like; cardiovascular agents such as coronary vasodilators and nitroglycerin; diagnostic agents; sympathomimetics; cholinomimetics; antimuscarinics; antispasmodics; hemostats to halt or prevent bleeding; muscle relaxants; adrenergic neuron blockers; antineoplastics; immunogenic agents; immunosuppressants; gastrointestinal drugs; diuretics; steroids; lipids; lipopolysaccharides; polysaccharides; and enzymes. It is also intended that combinations of medicinal agents may be used.

Other medicinal agents which may be included in the tissue support of the present disclosure include local anesthetics; non-steroidal antifertility agents; parasympathomimetic agents; psychotherapeutic agents; tranquilizers; sedative hypnotics; sulfonamides; vaccines; vitamins; antimalarials; anti-migraine agents; anti-parkinson agents such as L-dopa; anti-spasmodics; anticholinergic agents (e.g. oxybutynin); bronchodilators; alkaloids; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anticoagulants; anti-convulsants; antidepressants; anti-emetics; prostaglandins and cytotoxic drugs; estrogens; antibiotics; anti-fungals; anti-virals; and immunological agents.

Suitable antimicrobial agents which may be included as a medicinal agent in the tissue support of the present disclosure include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a medicinal agent in the tissue support of the present disclosure.

Examples of hemostat materials which can be employed include fibrin-based, collagen-based oxidized regenerated cellulose-based, and gelatin-based topical hemostats. Examples of commercially available hemostat materials include fibrinogen-thrombin combination materials sold under the trade designations CoStasis^{™} by Tyco Healthcare Group, LP, and Tisseel^{™} sold by Baxter International, Inc. Hemostats herein also include astringents, for example, aluminum sulfate, and coagulants.

Other examples-of suitable medicinal agents which may be included in the tissue support of the present disclosure include viruses and cells, peptides, polypeptides and proteins, analogs, muteins, and active fragments thereof, such as immunoglobulins, antibodies, cytokines (e.g. lymphokines, monokines, chemokines), blood clotting factors, hemopoietic factors, interleukins (IL-2, IL-3, IL-4, IL-6), interferons (β-IFN, (α-IFN and γ-IFN), erythropoietin, nucleases, tumor necrosis factor, colony stimulating factors (e.g., GCSF, GM-CSF, MCSF), insulin, anti-cancer and/or anti-tumor agents and tumor suppressors, blood proteins, gonadotropins (e.g., FSH, LH, CG, etc.), hormones and hormone analogs (e.g., growth hormone), vaccines (e.g., tumoral, bacterial and viral antigens); somatostatin; antigens; blood coagulation factors; growth factors (e.g., nerve growth factor, insulin-like growth factor); protein inhibitors, protein antagonists, and protein agonists; nucleic-acids, such as antisense molecules, DNA and RNA; oligonucleotides; and ribozymes.

A single medicinal agent may be utilized in the tissue support of the present disclosure or, in alternate embodiments, any combination of medicinal agents may be utilized in the tissue support of the present disclosure.

The medicinal agent may be disposed on a surface of the tissue support or impregnated into the tissue support.

Additionally, an enzyme may be added to tissue supports of the present disclosure to increase their rate of degradation. Suitable enzymes include, for example, peptide hydrolases such as elastase, cathepsin G, cathepsin E, cathepsin B, cathepsin H, cathepsin L, trypsin, pepsin, chymotrypsin, γ-glutamyltransferase (γ-GTP) and the like; sugar chain hydrolases such as phosphorylase, neuraminidase, dextranase, amylase, lysozyme, oligosaccharase and the like; oligonucleotide hydrolases such as alkaline phosphatase, endoribonuclease, endodeoxyribonuclease and the like. In some embodiments, where an enzyme is added, the enzyme may be included in a liposome or microsphere to control the rate of its release, thereby controlling the rate of degradation of the tissue support of the present disclosure. Methods for incorporating enzymes into liposomes and/or microspheres are with the purview of those skilled in the art.

The biocompatible tissue support has a number of advantageous properties. The resulting biocompatible tissue supports of the present disclosure are safe, possess enhanced adherence to tissue, are biodegradable, have enhanced hemostatic potential, have low cost, and are easy to prepare and use. By varying the selection of the polymer components, the strength and elasticity of the biocompatible tissue support can be controlled, as can the degradation time.

While the above description contains many specifics, these specifics should not be construed as limitations on the scope of the disclosure, but merely as exemplifications of embodiments thereof. Those skilled in the art will envision many other possibilities within the scope and spirit of the disclosure as defined by the claims appended hereto.

## Claims

1. A method comprising:
providing a sheet of a bioabsorbable polymeric material;
applying said sheet of bioabsorbable polymeric material to-a wound site;
applying energy to said sheet of bioabsorbable polymeric material;-and
shrinking said sheet of bioabsorbable polymeric material around said wound site.

2. The method of claim 1, wherein the bioabsorbable polymeric material is selected from the group consisting of glycolide, glycolic acid, lactide, lactic acid, caprolactone, dioxanone, trimethylene carbonate, dimethyl trimethylene carbonate, homopolymers thereof, copolymers thereof, and combinations thereof.

3. The method of claim 1, wherein the bioabsorbable polymeric material is selected from the group consisting of polycaprolactone, polylactic acid, polydioxanone, polytrimethylene carbonate, and combinations thereof.

4. The method of claim 1, wherein applying energy to said sheet of bioabsorbable polymeric material comprises energy selected from the group consisting of heat, light, ultraviolet radiation, magnetism, ultrasound, and combinations thereof.

5. The method of claim 1, wherein applying energy to said sheet of bioabsorbable polymeric material comprises heat from the body of a patient to which the tissue support is applied.

6. The method of claim 1, wherein applying energy to said sheet of bioabsorbable polymeric material comprises applying heat at a temperature from about 30° C to about 50° C for a period of time from about one second to about ten minutes.

7. The method of claim 1, wherein applying energy to said sheet of bioabsorbable polymeric material comprises applying light at a wavelength from about 400 nm to about 700 nm for a period of time from about one second to about ten minutes.

8. The method of claim 1, wherein applying energy to said sheet of bioabsorbable polymeric material comprises applying ultraviolet light at a wavelength from about 0.3 µm to about 3 nm for a period of time from about one second to about ten minutes.

9. The method of claim 1, wherein the bioabsorbable polymeric material has a thickness from about 0.002 inches to about 0.05 inches.

10. The method of claim 1, wherein the bioabsorbable polymeric material shrinks from about 5% to about 50% in area.

11. The method of claim 1, wherein the wound site comprises an anastomosis.

12. The method of claim 1, further comprising applying wound closure means to the wound site selected from the group consisting of sutures, staples, adhesives, and combinations thereof.

13. The method of claim 1, wherein the bioabsorbable polymeric material further comprises a medicinal agent.

14. The method of claim 13, wherein the medicinal agent is selected from the group consisting of antimicrobials, analgesics, antipyretics, anesthetics, antiepileptics, antihistamines, anti-inflammatory agents, cardiovascular agents, diagnostic agents, sympathomimetics, cholinomimetics, antimuscarinics, antispasmodics, hemostats, muscle relaxants, adrenergic neuron blockers, antineoplastics, immunogenic agents, immunosuppressants, gastrointestinal drugs, diuretics, steroids, lipids, lipopolysaccharides, polysaccharides, enzymes, local anesthetics, non-steroidal antifertility agents, parasympathomimetic agents, psychotherapeutic agents, tranquilizers, sedative hypnotics, sulfonamides, vaccines, vitamins, antimalarials, anti-migraine agents, anti-parkinson agents, anti-spasmodics, anticholinergic agents, bronchodilators, alkaloids, narcotics, non-narcotics, opioid receptor antagonists, anticoagulants, anti-convulsants, antidepressants, anti-emetics, prostaglandins, cytotoxic drugs, estrogens, antibiotics, anti-fungals, anti-virals, immunological agents, viruses, cells, peptides, polypeptides, proteins, muteins, vaccines, antigens, blood coagulation factors, growth factors, protein inhibitors, protein antagonists, protein agonists, nucleic acids, oligonucleotides, ribozymes, and combinations thereof.

15. The method of claim 1, wherein the bioabsorbable polymeric material possesses pores from about 2.5 mm to about 2.5x10⁻⁷ mm in diameter.

16. The method of claim 1, wherein the bioabsorbable polymeric material is fully degraded from about one day to about six months after application.

17. A wound tissue support comprising a sheet comprising a bioabsorbable polymeric material shrunk around a wound site, wherein the sheet is shrunk around the wound site by the application of energy to the bioabsorbable polymeric material.

18. The wound tissue support of claim 17, wherein the bioabsorbable polymeric material is selected from the group consisting of glycolide, glycolic acid, lactide, lactic acid, caprolactone, dioxanone, trimethylene carbonate, dimethyl trimethylene carbonate, homopolymers thereof, copolymers thereof, and combinations thereof.

19. The wound tissue support of claim 17, wherein the bioabsorbable polymeric material is selected from the group consisting of polycaprolactone, polylactic acid, polydioxanone, polytrimethylene carbonate, and combinations thereof.

20. The-wound tissue support of claim 17, wherein the sheet has a thickness from about 0.002 inches to about 0.05 inches.

21. The wound tissue support of claim 17, wherein the bioabsorbable polymeric material shrinks from about 5% to about 50% in area.

22. The wound tissue support of claim 17, wherein the wound site comprises an anastomosis.

23. The wound tissue support of claim 17, wherein the bioabsorbable polymeric material further comprises a medicinal agent.

24. The wound tissue support of claim 23, wherein the medicinal agent is selected from the group consisting of antimicrobials, analgesics, antipyretics, anesthetics, antiepileptics, antihistamines, anti-inflammatory agents, cardiovascular agents, diagnostic -agents, sympathomimetics, cholinomimetics, antimuscarinics, antispasmodics, hemostats, muscle relaxants, adrenergic neuron blockers, antineoplastics, immunogenic agents, immunosuppressants, gastrointestinal drugs, diuretics, steroids, lipids, lipopolysaccharides, polysaccharides, enzymes, local anesthetics, non-steroidal antifertility agents, parasympathomimetic agents, psychotherapeutic agents, tranquilizers, sedative hypnotics, sulfonamides, vaccines, vitamins, antimalarials, anti-migraine agents, anti-parkinson agents, anti-spasmodics, anticholinergic agents, bronchodilators, alkaloids, narcotics, non-narcotics, opioid receptor antagonists, anticoagulants, anti-convulsants, antidepressants, anti-emetics, prostaglandins, cytotoxic drugs, estrogens, antibiotics, anti-fungals, anti-virals, immunological agents, viruses, cells, peptides, polypeptides, proteins, muteins, vaccines, antigens, blood- coagulation factors, growth factors, protein inhibitors, protein antagonists, protein agonists, nucleic acids, oligonucleotides, ribozymes, and combinations thereof.

25. The wound tissue support of claim 17, wherein the bioabsorbable polymeric material possesses pores from about 2.5 mm to about 2.5x10⁻⁷ mm in diameter.

26. The wound tissue support of claim 17, wherein the bioabsorbable polymeric material is fully degraded from about one day to about six months after application.
